# EUROPÄISCHE PATENTSCHRIFT

(11) **EP 1 893 564 B1**
(45) Veröffentlichungstag und Bekanntmachung des Hinweises auf die Patenterteilung: **20.08.2008**
(21) Anmeldenummer: 06763725.6
(22) Anmeldetag: 14.06.2006
(51) Int. Cl.: C07C 263/20, C07C 265/10

(54) **VERFAHREN ZUR AUFARBEITUNG VON ISOCYANATADDUKTEN**
METHOD FOR PREPARING ISOCYANATE ADDUCTS
PROCEDE POUR TRAITER DES PRODUITS D'ADDITION D'ISOCYANATE

(30) Priorität: 15.06.2005 DE 102005027814
(43) Veröffentlichungstag der Anmeldung: 05.03.2008
(73) Patentinhaber: BASF SE, 67056 Ludwigshafen (DE)
(72) Erfinder: FIENE, Martin, 67150 Niederkirchen (DE); BÖHLING, Ralf, 64653 Lorsch (DE); STROEFER, Eckhard, 68163 Mannheim (DE); VOGEL, Herbert, 64569 Nauheim (DE); MESRI, Fatima, 76137 Karlsruhe (DE)
(86) Internationale Anmeldenummer: PCT/EP2006/063231
(87) Internationale Veröffentlichungsnummer: WO 2006/134137

(56) Entgegenhaltungen:
- WO-A-20/04108656
- FR-A- 2 378 748
- DATABASE WPI Section Ch, Week 200147 Derwent Publications Ltd., London, GB; Class A41, AN 2001-438602 XP002399176 & KR 2001 001 488 A (KOREA RES INST CHEM TECHNOLOGY) 5. Januar 2001 (2001-01-05) in der Anmeldung erwähnt

## Beschreibung

Gegenstand der Erfindung ist ein Verfahren zur Aufarbeitung von Isocyanataddukten, wie Polyurethanen, Rückständen aus der Isocyanatherstellung, insbesondere von Destillationsrückständen aus der Herstellung von Toluylendiisocyanat (TDI) oder Hexamethylendisocyanat (HDI).

Isocyanataddukte fallen in der Technik in großen Mengen als Abfall an. Beispiele sind Polyurethan-Schaumstoffe, hier beispielsweise Produktionsabfälle oder Schaumstoffe aus ausgemusterten Geräten, Kraftfahrzeugen oder Möbeln.

Eine weitere Gruppe von Isocyanataddukten sind Produktionsabfälle, insbesondere Destillationsrückstände, aus der Herstellung von Polyisocyanaten, insbesondere von Toluylendiisocyanat (TDI) oder Hexamethylendisocyanat (HDI). Besonders bei der Herstellung von TDI, einem der am meisten eingesetzten Polyisocyanate, fällt eine große Menge an Rückständen an.

TDI wird in großen Mengen zur Herstellung von Polyurethanen, insbesondere Polyurethan-Weichschaumstoffen, eingesetzt. Die Herstellung von TDI erfolgt zumeist durch Umsetzung von Toluylendiamin (TDA) mit Phosgen. Dieses Verfahren ist seit langem bekannt und vielfach in der Literatur beschrieben.

Üblicherweise wird dazu das TDA mit Phosgen in einer konventionellen zweistufigen Phosgenierung umgesetzt.

Dabei steht am Ende der Synthese üblicherweise ein Destillationsschritt, in dem das TDI von schwersiedenden Nebenprodukten abgetrennt wird. Aus verfahrenstechnischen Gründen; beispielsweise um die Pumpfähigkeit des Rückstands zu gewährleisten, kann der Rückstand noch bis zu 70 %, bevorzugt bis zu 50 % besonders bevorzugt bis zu 30 % TDI enthalten. Es besteht also für die heutigen "World-Scale-Anlagen" von bis zu mehreren 100.000 t Jahreskapazität ein erheblicher wirtschaftlicher Anreiz, diesen Rückstand stofflich zu verwerten.

Eine häufig praktizierte Möglichkeit, zumindest einen Teil des in dem Destillationsrückstand enthaltenen TDI zu gewinnen, besteht in der weiteren Entfernung des TDI aus dem Rückstand, beispielsweise mittels eines Extruders. Geeignete Apparate sind beispielsweise die sogenannten List-Trockner. Hierbei handelt es sich um spezielle Schaufeltrockner der Firma List, die häufig in der Isocyanatproduktion Verwendung finden. Hierdurch kann die TDI-Menge im Destillationsrückstand signifikant erniedrigt werden. Es fällt jedoch auch bei diesem Prozess noch ein in der Regel fester Rückstand an, durch den die Ausbeute des Verfahrens gesenkt wird. Dieser wird bislang zumeist verbrannt.

Eine alternative Möglichkeit der Verwertung der Destillationsrückstände ist ihre stoffliche Verwertung. Dazu sind verschiedene Verfahren bekannt.

Eine solche Möglichkeit der Verwertung ist die Umsetzung des Rückstandes mit Wasser, die sogenannte Hydrolyse. Derartige Verfahren sind vielfach beschrieben. Die Hydrolyse des Rückstandes wird durch Basen bzw. Säuren begünstigt. Auch Amine begünstigen die Hydrolyse. Die Hydrolyse kann dazu genutzt werden, den TDI-Destillationsrückstand zu Denaturieren, wie beispielsweise in US-A-4,091,009 beschrieben. Eine weitere Möglichkeit ist die Rückgewinnung von TDA, das dann wieder mit Phosgen zu TDI umgesetzt werden kann. Derartige Verfahren werden beispiels-weise in DE-A-29 42 678, JP-A-5 8201 751 und DE-A-19 62 598 beschrieben.

In DE-A-27 03 313 wird ein Hydrolyseverfahren beschrieben, das sowohl diskontinuierlich in einem Autoklaven als auch kontinuierlich in einem Röhrenreaktor durchgeführt werden kann. Die Hydrolyse des festen TDI-Rückstandes wird mit wässriger Ammoniaklösung, Lösungen von primärem oder sekundären Aminen in Wasser oder wässriger TDA-Lösung durchgeführt.

US-A-4,654,443 beschreibt ein Hydrolyseverfahren, bei dem in einem ersten Verfahrensschritt der TDI-Rückstand mit TDA zu einem Feststoff umgesetzt und in einem zweiten Schritt dieses Zwischenprodukt mit Wasser hydrolysiert wird. Nachteilig ist hier, dass das Verfahren zwei Verfahrensschritte umfasst, und dass TDA dem Reaktionsgemisch zugesetzt werden muss. Außerdem kommt es zu einer starken Ausbildung von Feststoffen.

In WO 99/65868 wird ein kontinuierliches beziehungsweise halbkontinuierliches Verfahren zur Hydrolyse von Destillationsrückständen in einem rückvermischten Reaktor beschrieben. Durch diese Verfahrensführung kann die Ausbildung von Feststoffen verhindert werden.

In JP-A-151 270/97 wird ein Verfahren zur Hydrolyse von TDI-Rückständen mit überkritischem oder heißem Hochdruckwasser beschrieben. Nachteilig bei diesem Verfahren ist der sehr hohe Druck, der die Verwendung spezieller Apparate notwendig macht, sowie die Korrosionsprobleme, die sich aus der Verwendung von überkritischem Wasser und den in den Rückständen häufig noch vorhandenen chlorhaltigen Nebenprodukten ergeben. Außerdem muss mit einem hohen Wasserüberschuss gearbeitet werden.

WO 04/108656 beschreibt die Zersetzung von pulverisierten TDI-Rückständen mit Wasser unterhalb des kritischen Punkts in Gegenwart von Katalysatoren. Auch bei diesem Verfahren ist die Bildung von Feststoffen nicht auszuschließen.

KR 383217 beschreibt ein Verfahren zur Gewinnung von TDA aus Destillationsrückständen durch Umsetzung mit wässriger Ammoniaklösung. Dabei wird ein Destillationsrückstand eingesetzt, der einen Gehalt an freiem TDI von unter 1000 ppm aufweist. Die Umsetzung kann unterhalb des kritischen Punkts, im Bereich des kritischen Punkts oder oberhalb des kritischen Punkts von Wasser durchgeführt werden.

Nachteilig bei allen beschriebenen Verfahren zur Aufarbeitung von Destillationsrückständen aus der TDI-Herstellung ist, dass es durch die Reaktion des Rückstands mit Wasser zur Bildung von Kohlendioxid und damit zu einem zusätzlichen Druckaufbau im Reaktor kommt. Will man das bei der Aufarbeitung gebildete TDA in den Produktionsprozess zurückführen, muss man im Anschluss an die Hydrolyse das Wasser wieder aufwändig aus der Reaktionsmischung entfernen, da Wasserspuren in der Phosgenierung bereits im ppm-Bereich zu Korrosion und Harnstoffbildung führen. In der Regel entfernt man das Wasser durch Destillation. Bei diesem Schritt werden erneut hochmolekulare Rückstände gebildet, die zu Ausbeuteverlusten an zuvor gebildetem TDA führen. Ein weiterer Nachteil bei der Hydrolyse von TDI-Rückständen mit einem wasserhaltigen Reaktionsgemisch besteht in der geringeren Selektivität. Die bei der Reaktion gebildeten Amine reagieren wie in dem folgenden Schema gezeigt in einer Folgereaktion mit dem Wasser zu sogenannten Aminokresolen.

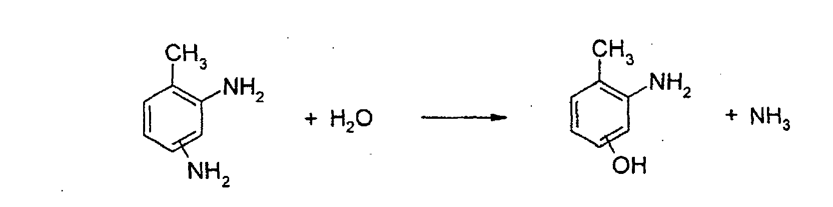

Auch dieser Prozess führt zu Ausbeuteverlusten an TDA.

Aufgabe der Erfindung war es daher, ein Verfahren zur Aufarbeitung von Isocyanataddukten, wie Polyurethanen und Destillationsrückständen aus der Herstellung von Isocyanaten, insbesondere aus der TDI-Herstellung, zu entwickeln, das einfach und mit hohen Ausbeuten zu betreiben ist.

Die Aufgabe konnte überraschenderweise gelöst werden, indem die Isocyanataddukte mit reinem Ammoniak umgesetzt werden.

Gegenstand der Erfindung ist demzufolge ein Verfahren zur Aufarbeitung von Isocyanataddukten, umfassend die Schritte:
a) Umsetzung der Isocyanataddukte mit reinem Ammoniak,
b) Aufarbeitung der in Schritt a) entstehenden Umsetzungsprodukte,
c) Rückführung der gebildeten Amine in die Isocyanatproduktion.

Bei den Isocyanataddukten kann es sich um Umsetzungsprodukte der Isocyanate mit sich selbst oder mit Verbindungen mit funktionellen Gruppen, die mit Isocyanatgruppen reagieren können; handeln.

Beispiele für Isocyanataddukte sind kompakte oder geschäumte Polyurethane, Umsetzungsprodukte von Isocyanaten mit sich selbst, wie Isocyanurate, Uretonimine, Uretdione, Cabodiimide sowie oligomere und polymere Umsetzungsprodukte der Isocyanate. Insbesondere können für das erfindungsgemäße Verfahren Produktionsrückstände aus der Herstellung von Isocyanaten, insbesondere flüssige oder feste Destillationsrückstände aus der TDI- und/oder HDI-Herstellung, eingesetzt werden.

Bei Einsatz von festen Produktionsrückständen oder Polyurethanen werden diese zunächst zerkleinert, vorzugsweise auf eine Teilchengröße < 100 mm, bevorzugt < 10 mm besonders bevorzugt < 1 mm. Bei Polyurethan-Schaumstoffen erfolgt vorzugsweise eine Kompaktierung, beispielsweise durch Pressen oder Mahlen.

Bei Einsatz von flüssigen Produktionsrückständen aus der Herstellung von Isocyanaten werden diese zumeist in flüssigem Zustand dem erfindungsgemäßen Verfahren unterzogen.

Die Destillationsrückstände aus der TDI-Herstellung können direkt aus der Anlage dem erfindungsgemäßen Verfahren zugeführt werden. In einer Ausführungsform des Verfahrens können die Destillationsrückstände in flüssigem Zustand zu dem erfindungsgemäßen Verfahren gepumpt werden. Dabei ist jedoch sicherzustellen, dass die Rückstände nicht erstarren, da sie dann nicht mehr verflüssigt werden können. Es ist auch möglich, die Rückstände erstarren zu lassen und in zerkleinerter Form, beispielsweise als Pulver oder Pellets, dem erfindungsgemäßen Verfahren zuzuführen. Dabei haben die zerkleinerten Rückstände vorzugsweise die oben genannte Teilchengröße.

In einer bevorzugten Ausführungsform des erfindungsgemäßen Verfahrens wird aus den Destillationsrückständen der Isocyanatherstellung, insbesondere der Herstellung von TDI- und/oder HDI, vor der Umsetzung mit dem Ammoniak das freie Isocyanat abgetrennt. Dies kann beispielsweise mittels Dünnschichtdestillation erfolgen, vorzugsweise jedoch mittels eines "List-Trockners".

Der Destillationsrückstand hat nach dieser Behandlung vorzugsweise noch einen Gehalt an freiem Isocyanat von maximal 5000 ppm (g pro g). Er besteht hauptsächlich aus oligomeren und polymeren Isocyanat- bzw. Carbodiimid-Addukten. Die genaue Zusammensetzung hängt jeweils stark von den zuvor gewählten Reaktionsbedingungen ab.

Der Vorteil dieser Verfahrensweise besteht hauptsächlich darin, dass das bereits im Verfahren gebildete Isocyanat nicht noch einmal stofflich umgewandelt wird, außerdem wird die Einsatzmenge für das erfindungsgemäße Verfahren reduziert. Darüber hinaus können im Falle von TDI in dem Listtrockner TDI-Dimere zum TDI zurückgespalten werden.

Je nach Größe der in der jeweiligen Anlage anfallenden Stoffströme kann es aus wirtschaftlicher Sicht auch sinnvoller sein, die Destillationsrückstände ohne weitere Abtrennung der monomeren Isocyanate direkt einer Ammonolyse zu unterziehen.

Es ist prinzipiell auch möglich, zur Verbesserung der Handhabbarkeit der Isocyanatrückstände diese in einem geeigneten, organischen Lösemittel, das mit dem Rückstand nicht reagiert, wie z.B. Toluol, Monochlorbenzol, Dichlorbenzol und anderen, aufzunehmen. Diese Ausführungsform ist jedoch nicht bevorzugt, da das Lösungsmittel in einem gesonderten Schritt abgetrennt werden muss.

Die Ammonolyse kann sowohl kontinuierlich als auch diskontinuierlich durchgeführt werden. Die Entscheidung hierüber hängt vor allem von der Menge der in der jeweiligen Isocyanatproduktion anfallenden Rückstände ab.

Die Umsetzung der Isocyanataddukte mit Ammoniak wird so durchgeführt, dass der Ammoniak im überkritischen Zustand oder in der Nähe des kritischen Punkts vorliegt. Dementsprechend wird die Umsetzung vorzugsweise bei einer Temperatur im Bereich zwischen 100 und 500°C, bevorzugt 100 bis 400°C besonders bevorzugt 100 bis 250°C und einem Druck im Bereich zwischen 100 und 500 bar, bevorzugt 100 bis 400 bar und besonders bevorzugt 100 und 380 bar durchgeführt.

Das Ammoniak muss hinsichtlich der zu spaltenden Bindung mindestens äquimolar vorliegen. Vorzugsweise wird es in einem mindestens 10 %-igen molaren Überschuss eingesetzt. Da die Zusammensetzung der Rückstände stark von den Reaktionsbedingungen im Herstellungsverfahren abhängt und analytisch nicht genau gefasst werden kann, erfolgt die Mengenangabe des Ammoniaks im folgenden in Gew.-%. Vorzugsweise liegt der Ammoniakanteil der Ausgangskomponenten des erfindungsgemäßen Verfahrens im Bereich von 10 Gew.-% bis zu 90 Gew.-%, bevorzugt 30 - 70 Gew.-%; bezogen auf das Reaktionsgemisch.

Die Reaktion kann in Rohrreaktoren, in Kesseln oder in Rührkesselkaskaden durchgeführt werden. Die Verweilzeit liegt vorzugsweise zwischen 30 Sekunden und 5 Stunden, bevorzugt zwischen 1 Minute und 1 Stunde.

Die Aufarbeitung des Umsetzungsprodukts aus dem erfindungsgemäßen Verfahren erfolgt allgemein durch Abtrennung der leicht flüchtigen Bestandteile, insbesondere des überschüssigen Ammoniaks, Auftrennung der Spaltprodukte und deren Aufarbeitung. Bei den Spaltprodukten handelt es sich zum einen um die den Isocyanaten zugrundeliegenden Amine und, im Falle der Ammonolyse von Polyurethanen, zusätzlich um die um die dem Polyurethan zugrundeliegenden Alkoholkomponenten.

Das Umsetzungsprodukt des erfindungsgemäßen Verfahrens wird dem Reaktor zumeist kontinuierlich entnommen und aufgearbeitet. Beim bevorzugten lösungsmittelfreien Verfahren ist das Umsetzungsprodukt bei vollständiger Umsetzung einphasig.

Die Aufarbeitung soll am Beispiel des TDI-Rückstands näher beschrieben werden. In ähnlicher Weise erfolgt auch die Aufarbeitung der Umsetzungsprodukte aus anderen Verfahren, wie beispielsweise HDI.

Zunächst erfolgt die Abtrennung des im Überschuss eingesetzten Ammoniaks, vorzugsweise durch Flash und/oder Strippen, und anschließend die Abtrennung des TDA, vorzugsweise mittels Destillation oder Kristallisation. Das abgetrennte Ammoniak kann wieder verdichtet und in die Ammonolyse zurückgeführt oder energetisch genutzt werden.

Das abgetrennte TDA kann nach entsprechender Reinigung und Aufarbeitung entweder dem Phosgenierungsreaktor des TDI-Prozesses zugesetzt oder dem Reaktionsgemisch, das bei der TDA-Herstellung durch Hydrierung von Dinitrotouol den Hydrierreaktor verlässt, vor der Aufarbeitung zum reinen TDA zugeführt werden. Die letztgenannte Verfahrensvariante hat den Vorteil, dass der Aufarbeitungsschritt nach der Ammonolyse vereinfacht oder gegebenenfalls ganz eingespart werden kann. Die Nebenprodukte der Ammonolyse können dann im Verfahrensschritt der TDA-Aufarbeitung mit dem TDA-Teer aus dem Verfahren ausgeschleust werden.

Nach der Ammonolyse noch verbleibende Rückstände können auf übliche Weise entsorgt werden, beispielsweise durch Verbrennung. Bei den verbleibenden Rückständen handelt es sich beispielsweise um nicht umgesetzte Edukte, um Guanidine oder, insbesondere bei der Ammonolyse von Polyurethanen, um Harnstoffe. Diese Nebenprodukte können vor der Rückführung der Amine in das Verfahren zur Herstellung der Polyisocyanate abgetrennt werden. Es ist jedoch auch möglich, das TDA ungereinigt dem aus der Hydrierung entnommenen TDA-Strom zuzuführen. In der anschließenden Reinigung werden die enthaltenen Verunreinigungen mit entfernt.

Beim Einsatz von Polyurethanschäumen in der Ammonolyse kann der Reaktionsaustrag nach der Abtrennung des Ammoniaks ohne weitere Aufarbeitung oder nach der Abtrennung von Nebenprodukten auch zur Herstellung von Polyetheralkoholen durch Anlagerung von Alkylenoxiden verwendet werden. Es ist natürlich auch möglich, das gereinigte TDA mit Alkylenoxiden zu Polyetheralkoholen umzusetzen.

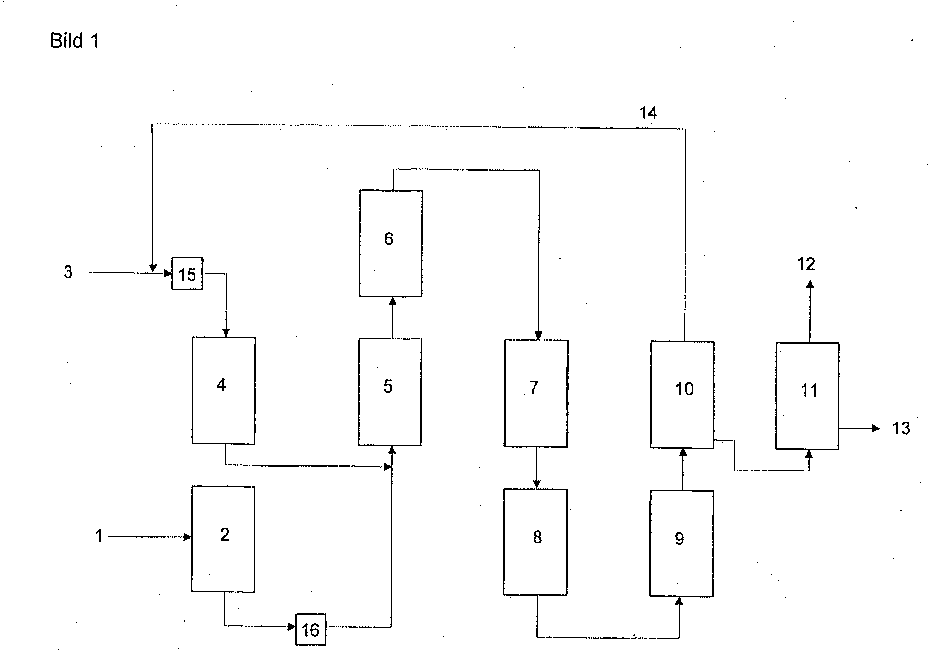

Ein schematisches Verfahrensfließbild für die Umsetzung eines flüssigen Isocyanatrückstands wird in Bild 1 gezeigt. Dabei wird der Produktionsrückstand 1 entweder zunächst in einen Pufferbehälter 2 oder direkt mit einer Pumpe 16 zu einem statischen Mischer 5 gefahren. Von dort wird er mit einer Pumpe 16 zu einem statischen Mischer 5 gefördert und dabei auf das gewünschte Druckniveau gebracht. Im statischen Mischer wird er mit Ammoniak gemischt. Dieser Ammoniak setzt sich zusammen aus einem Feedstrom 3 und optional einem Recyclestrom 14. Beide Ströme werden mit dem Verdichter 15 auf den gewünschten Druck verdichtet und bei Bedarf in einem Pufferbehälter 4 zwischengelagert. Beide Feedströme können prinzipiell bereits vor dem statischen Mischer auf Reaktionstemperatur vorgeheizt werden. Ergänzend oder auch alternativ folgt ein weiterer Wärmetauscher 6 hinter dem statischen Mischer, ehe das Gemisch in einem Reaktor 7 umgesetzt wird. Anschließend wird der Reaktionsaustrag in einem Kühler 8 abgekühlt und an einer Druckhaltevorrichtung 9 entspannt. Der Kühler 8 kann prinzipiell auch als Wärmeverbundapparat ausgeführt sein, in dem z.B. der Kreislaufstrom 14 vorgeheizt wird. In einem Abscheider 10 wird die flüssige TDAhaltige Phase vom überschüssigen Ammoniak abgetrennt. Die Ammoniakphase wird als Kreislaufstrom 14 zurückgefahren und vor dem Verdichter 15 wieder eingespeist. Das gebildete Amin wird in einer nachgeschalteten Kolonne 11 von nicht umgesetztem Rückstand 13 abdestilliert. Das so gereinigte Amin kann in einer Isocyanatanlage wieder eingesetzt werden. Der Rückstand 13 wird entsorgt.

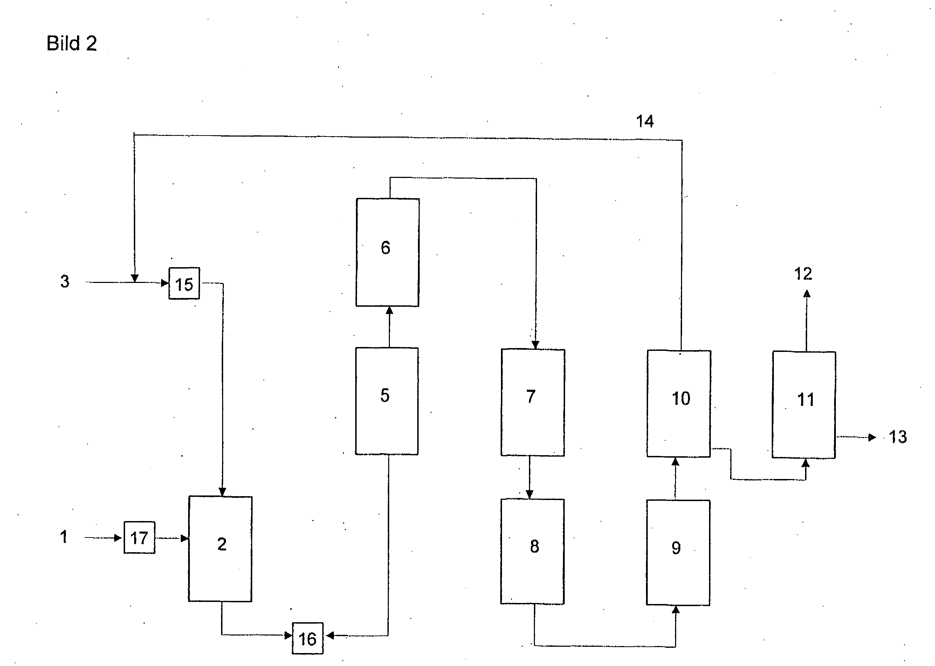

Bild 2 zeigt ein schematisches Verfahrensfließbild für die Umsetzung eines festen Isocyanatrückstands. Das Verfahren ähnelt dem zur Aufarbeitung der flüssigen Rückstände. Allerdings muss der feste Rückstand zunächst mit einer Mühe 17 zerkleinert werden. Auf den Behälter 4 kann man hier verzichten.

Bei der Umsetzung von Polyurethanrückständen nach dem erfindungsgemäßen Verfahren werden die bei der Reaktion gebildeten Polyole nach der Abtrennung des Amins entweder direkt ohne Abtrennung der nicht umgesetzten Edukte zur Herstellung von Polyurethanen verwendet oder gereinigt, beispielsweise durch Destillation oder Extraktion. Danach können sie wieder zur Herstellung von Polyurethanen eingesetzt werden.

Das Verfahren hat gegenüber der Hydrolyse von Isocyanataddukten erhebliche Vorteile. So kann das Verfahren bei niedrigeren Drücken und Temperaturen betrieben werden, da der kritische Punkt von Ammoniak niedriger ist als der von Wasser und von Gemischen aus Wasser und Ammoniak.

Da bei dem erfindungsgemäßen Verfahren kein Wasser eingesetzt und gebildet wird, kann die aufwändige Entfernung des Wassers aus dem Reaktionsprodukt entfallen. Dies ist besonders bedeutsam, da bereits Spuren von Wasser bei der Phosgenierung der Amine zu Betriebsstörungen infolge von Feststoffbildung und/oder Korrosion führen können.

Darüber hinaus wird im Falle der Umsetzung von Isocyanataddukten auf Basis von TDI eine höhere Selektivität zum TDA erzielt, da es nicht zur Bildung von Aminokresolen durch Folgereaktionen mit Wasser kommen kann.

Weiterhin entsteht bei der Ammonolyse kein Kohlendioxid, das zu einem Druckanstieg im Reaktor führen würde.

Das Verfahren soll an den nachstehenden Beispielen näher beschrieben werden.

Die Apparatur besteht aus einem 5 ml Autoklav aus Inconel 625, der mit einem Thermoelement, einer Bestscheibe (Bestdruck 400 bar), einem Manometer und einem Hochdruckventil ausgestattet ist. Die Apparatur kann mit einer Ölpumpe evakuiert und unter inertgas befüllt und entlehrt werden.

Bei einem typischen Ansatz wurde der Reaktor mit 0,07 g umzusetzendem Edukt gemäß Tabelle gefüllt. Anschließend wurde Ammoniak einkondensiert. Die Beheizung des Reaktors erfolgte in einem Ofen. Nach 30 min wurde der Reaktor aus dem Ofen genommen, abgekühlt und entspannt. Die Ausbeuten wurden mittels kalibrierter Gaschromatographie (GC)-Messungen ermittelt. Die eingesetzten Ausgangsprodukte, die Drücke, die Temperaturen bei der Umsetzung und die GC-Ausbeuten sind in nachfolgender Tabelle dargestellt.

| Beispiele: | | | | | |
|---|---|---|---|---|---|
| Edukt | Oligomeres Carbodiimid auf Basis TDI | Listrückstand (TDI-Gehalt <1 Gew.-%) | Listrückstand (TDI-Gehalt <1 Gew.-%) | Listrückstand (TDI-Gehalt <1 Gew.-%) (V) | PU-Schaum |
| Temp. [°C] | 185 | 148 | 137 | 86 | 150 |
| Druck [bar] | 200 | 380 | 180 | 320 | 176 |
| Gew.-% NH₃ im Gesamtgemisch | 60 | 90 | 90 | 90 | 75 |
| TDA-Ausbeute | >90% | >90 | 86 | 20 | 84 |

| | | | | | |
|---|---|---|---|---|---|
| V - Vergleichsbeispiel mit nichtkritischem Ammoniak | | | | | |

Bei dem PU-Schaum handelte es sich um einen Polyurethan-Blockweichschaumstoff auf Basis von TDI und einem dreifunktionellen Polyetheralkohol auf Basis von Glyzerin, Ethylenoxid und Propylenoxid mit einer Hydroxylzahl von 36 mgKOH/g.

## Patentansprüche

1. Verfahren zur Aufarbeitung von Isocyanataddukten, umfassend die Schritte
a) Umsetzung der Isocyanataddukte mit reinem Ammoniak,
b) Aufarbeitung der in Schritt a) entstehenden Umsetzungsprodukte,
c) Rückführung der gebildeten Amine in die Isocyanatproduktion,
wobei bei dem Verfahren kein Wasser eingesetzt wird.

2. Verfahren nach Anspruch 1, **dadurch gekennzeichnet, dass** die Umsetzung so geführt wird, dass das Ammoniak im überkritischen Zustand oder in der Nähe des kritischen Punkts vorliegt.

3. Verfahren nach Anspruch 1, **dadurch gekennzeichnet, dass** das Ammoniak hinsichtlich der zu spaltenden Bindung mindestens äquimolar vorliegt.

4. Verfahren nach Anspruch 1, **dadurch gekennzeichnet, dass** die Umsetzung bei einer Temperatur im Bereich zwischen 100 und 500°C und einem Druck im Bereich zwischen 100 und 500 bar durchgeführt wird.

5. Verfahren nach Anspruch 1, **dadurch gekennzeichnet, dass** die Isocyanataddukte Umsetzungsprodukte der Isocyanate mit sich selbst oder mit Verbindungen mit funktionellen Gruppen, die mit Isocyanatgruppen reagieren können, sind.

6. Verfahren nach Anspruch 1, **dadurch gekennzeichnet, dass** die Isocyanataddukte ausgewählt sind aus der Gruppe, enthaltend kompakte oder geschäumte Polyurethane, Isocyanurate, Uretonimine, Uretdione, Cabodiimide sowie oligomere und polymere Umsetzungsprodukte der Isocyanate.

7. Verfahren nach Anspruch 1, **dadurch gekennzeichnet, dass** die Isocyanataddukte flüssige oder feste Destillationsrückstände aus der Herstellung von Isocyanaten sind.

8. Verfahren nach Anspruch 1, **dadurch gekennzeichnet, dass** die Isocyanataddukte flüssige oder feste Destillationsrückstände aus der TDI-Herstellung sind.

9. Verfahren nach Anspruch 1, **dadurch gekennzeichnet, dass** die festen Isocyanataddukte vor der Umsetzung zerkleinert werden.

10. Verfahren nach Anspruch 1, **dadurch gekennzeichnet, dass** die festen Isocyanataddukte vor der Umsetzung auf eine Teilchengröße < 100 mm zerkleinert werden.

11. Verfahren nach Anspruch 1, **dadurch gekennzeichnet, dass** die Umsetzung in Rohrreaktoren, in Kesseln oder in Rührkesselkaskaden durchgeführt wird.

12. Verfahren nach Anspruch 1, **dadurch gekennzeichnet, dass** aus den Destillationsrückständen der TDI-Herstellung vor der Umsetzung mit dem Ammoniak das freie TDI abgetrennt wird.

## Claims

1. A process for working up isocyanate adducts, comprising the steps of
a) reacting the isocyanate adducts with pure ammonia,
b) working up the reaction products obtained in step a),
c) recycling the amines formed into the isocyanate production,
no water being used in the process.

2. The process according to claim 1, wherein the reaction is conducted in such a way that ammonia is present in the supercritical state or close to the critical point.

3. The process according to claim 1, wherein the ammonia is present in an at least equimolar amount with respect to the bond to be cleaved.

4. The process according to claim 1, wherein the reaction is carried out at a temperature in the range between 100 and 500°C and a pressure in the range between 100 and 500 bar.

5. The process according to claim 1, wherein the isocyanate adducts are reaction products of the isocyanates with themselves or with compounds having functional groups which can react with isocyanate groups.

6. The process according to claim 1, wherein the isocyanate adducts are selected from the group comprising compact or foamed polyurethanes, isocyanurates, uretonimines, uretdiones, carbodiimides, and also oligomeric and polymeric reaction products of the isocyanates.

7. The process according to claim 1, wherein the isocyanate adducts are liquid or solid distillation residues from the preparation of isocyanates.

8. The process according to claim 1, wherein the isocyanate adducts are liquid or solid distillation residues from TDI preparation.

9. The process according to claim 1, wherein the solid isocyanate adducts are comminuted before the reaction.

10. The process according to claim 1, wherein the solid isocyanate adducts are comminuted before the reaction to a particle size of < 100 mm.

11. The process according to claim 1, wherein the reaction is carried out in tubular reactors, in tanks or in stirred tank batteries.

12. The process according to claim 1, wherein the free TDI is removed from the distillation residues of TDI preparation before the reaction with the ammonia.

## Revendications

1. Procédé servant à traiter des produits d'addition d'isocyanate, comprenant les étapes de :
a) transformation des produits d'addition d'isocyanate avec de l'ammoniac pur,
b) traitement des produits de la transformation obtenus à l'étape a),
c) récupération des amines formées dans la production d'isocyanate,
de l'eau n'étant utilisée dans le procédé.

2. Procédé selon la revendication 1, **caractérisé en ce que** la transformation est menée de telle sorte que l'ammoniac se trouve à l'état hypercritique ou proche du point critique.

3. Procédé selon la revendication 1, **caractérisé en ce que** l'ammoniac concernant la liaison à scinder est présent au moins en quantité équimolaire.

4. Procédé selon la revendication 1, **caractérisé en ce que** la transformation est effectuée à une température comprise dans la plage entre 100 et 500 °C et à une pression comprise dans la plage entre 100 et 500 bar.

5. Procédé selon la revendication 1, **caractérisé en ce que** les produits d'addition d'isocyanate sont des produits de transformation des isocyanates avec lui-même ou avec des composés comprenant des groupes fonctionnels qui peuvent réagir avec les groupes d'isocyanate.

6. Procédé selon la revendication 1, **caractérisé en ce que** les produits d'addition d'isocyanate proviennent du groupe contenant les polyuréthannes, les isocyanurates, les urétonimines, les uretdiones, les carbodiimides compacts ou expansés, ainsi que les produits de transformation oligomériques ou polymériques des isocyanates.

7. Procédé selon la revendication 1, **caractérisé en ce que** les produits d'addition d'isocyanate sont des résidus liquides ou solides de distillation issus de la fabrication d'isocyanates.

8. Procédé selon la revendication 1, **caractérisé en ce que** les produits d'addition d'isocyanate sont des résidus liquides ou solides de distillation issus de la fabrication de TDI.

9. Procédé selon la revendication 1, **caractérisé en ce que** les produits d'addition d'isocyanate solides sont réduits avant la transformation.

10. Procédé selon la revendication 1, **caractérisé en ce que** les produits d'addition d'isocyanate solides sont réduits avant la transformation à une taille de particules < 100 mm.

11. Procédé selon la revendication 1, **caractérisé en ce que** la transformation est réalisée dans des réacteurs tubulaires, dans des cuves ou dans des réacteurs à cuve à agitation.

12. Procédé selon la revendication 1, **caractérisé en ce que** le TDI libre est séparé des résidus de distillation de la fabrication de TDI avant la transformation avec l'ammoniac.
